# EUROPÄISCHE PATENTSCHRIFT

(11) **EP 2 575 729 B1**
(45) Veröffentlichungstag und Bekanntmachung des Hinweises auf die Patenterteilung: **11.02.2015**
(21) Anmeldenummer: 11743957.0
(22) Anmeldetag: 16.05.2011
(51) Int. Cl.: A61H 33/00, A61H 37/00

(54) **LIEGE ZUR PHYSIOTHERAPEUTISCHEN BEHANDLUNG DES MENSCHLICHEN KÖRPERS**
BED FOR THE PHYSIOTHERAPEUTIC TREATMENT OF THE HUMAN BODY
COUCHETTE POUR LE TRAITEMENT PHYSIOTHÉRAPEUTIQUE DU CORPS HUMAIN

(30) Priorität: 27.05.2010 DE 202010007306 U
(43) Veröffentlichungstag der Anmeldung: 10.04.2013
(73) Patentinhaber: Weirich, Birgit, 79618 Rheinfelden (DE)
(72) Erfinder: Weirich, Birgit, 79618 Rheinfelden (DE)
(74) Vertreter: Ebert, Jutta
(86) Internationale Anmeldenummer: PCT/DE2011/001072
(87) Internationale Veröffentlichungsnummer: WO 2011/147402

(56) Entgegenhaltungen:
- WO-A1-2010/005274
- US-A1- 2007 000 043

## Beschreibung

Die Erfindung betrifft eine Liege zur physiotherapeutischen Behandlung des menschlichen Körpers mit einer auf einem Unterbau ruhenden, erwärmbaren Liegefläche.

Für unterschiedliche physiotherapeutische Behandlungen des menschlichen Körpers sowohl im Trocken- wie im Nassbereich, wie Massage-Behandlungen, Kosmetik-Behandlungen, Aromatherapie-Behandlungen, unterschiedliche Körperpackungen, wie Fango-, Algen- oder Moorpackungen, Anti-Cellulite-Behandlungen, Seifenbürstenmassagen und Dampfbehandlungen existieren bereits unterschiedliche Behandlungsliegen, auf denen eine zu behandelnde Person in einer möglichst entspannten Haltung Platz nehmen kann, um sich auf eine bestimmte Weise behandeln zu lassen. In der einfachsten Form besteht eine solche Liege aus einem langgestreckten, tischartigen Untergestell mit einer gepolsterten, matratzenartigen Auflage, die mit einem Überzug bedeckt ist, der nach jeder Behandlung ausgewechselt und gewaschen werden muss, um hygienischen Mindestanforderungen gerecht werden zu können. Damit der Kopf bei einer Behandlung in einer entspannten Stellung gehalten werden kann, ist an solchen Liegen häufig eine Kopfteil vorgesehen, das im einfachsten Fall als Abschrägung auf der einen Schmalseite der matratzenähnlichen Auflage ausgebildet sein kann oder aus einer an der einen Schmalseite der Liege angebrachten, meist in ihrer Neigung verstellbaren Kopfstütze bestehen kann. Häufig ist in einem solchen Kopfteil eine Aussparung für das Gesicht einer zu behandelnden Person vorgesehen, damit diese auch in Bauchlage eine bequeme, entspannte Stellung einnehmen kann.

Durch die DE 299 05 089 U1 ist eine solche Liege bekannt, bei der das Kopfteil relativ zum Rumpfteil zusätzlich schwenkbar, rotierbar und in im wesentlichen horizontaler Richtung verschiebbar ist, um auch an Personen mit bestimmten Behinderungen oder Haltungsschäden angepasst werden zu können. Die Liege ist insgesamt höhenverstellbar.

Solche einfachen Liegen eignen sich im Grunde nur für Behandlungen im Trockenbereich, wie Massagebehandlungen, Kosmetikbehandlungen usw.

Es werden zwar auch zeltartige Hauben angeboten, die über eine solche einfache Liege und die darauf liegende Person gestülpt werden können und die dann am Umfang der Liege dicht genug abschließen, damit Dampf in die Haube eingeleitet werden kann und so auch Dampfbehandlungen auf einer solchen einfachen Liege möglich werden. Von Nachteil ist, dass bei einer solchen Dampfbehandlung die textilen Teile der matratzenartigen Auflage und der Überzug stark durchfeuchtet werden, was noch während der Behandlung mit der Zeit ein unangenehmes Gefühl bei der behandelten Person hervorrufen kann. Nicht nur der Überzug sondern auch die Auflage müssen anschließend an eine Dampfbehandlung komplett ausgewechselt, gewaschen und getrocknet werden. Besonders die Durchfeuchtung der matratzenähnlichen Auflage kann mit der Zeit zu hygienischen Problemen führen, etwa wenn die Trocknung nicht immer intensiv genug durchgeführt wird.

Die DE 30 33 419 A1 schlägt eine tischförmige Behandlungsliege speziell für Wärmebehandlungen vor, bei der eine Wärme spendende Vorrichtung in oder unter der Liegefläche in die Behandlungsliege integriert ist; nach der beschriebenen Ausführungsform wird ein Wärmespender zwischen der gepolsterten Auflage und dem Auflagenbezug eingelegt.

Behandlungen im Nassbereich, wie die verschiedenen Körperpackungen oder Seifenbürstenmassagen werden meistens in entsprechenden Wannen durchgeführt, in denen die zu behandelnden Personen liegend Platz nehmen können. Die behandelnde Person muss dabei während der Behandlung immer wieder eine anstrengende, besonders das Rückgrat belastende Haltung einnehmen. Die Wannen müssen nach jeder Behandlung von einer Person sorgfältig und in gebückter, belastender Haltung gereinigt werden. Auch Matten, die die Rutschgefahr in solchen Wannen mindern sollen, müssen immer hygienisch sauber gehalten werden.

Die DE10 2005 059 389 A1 schlägt eine Vorrichtung vor, bei der Anwendungen auf einer Folie vorgenommen werden, die über eine Wanne gespannt ist und auch im belasteten Zustand, also wenn eine Person auf der Folie liegend Platz genommen hat, nur wenig durchhängen soll und so eine schalen- oder tellerförmige Auflagefläche bilden soll. Die Folie kann über am Wannenrand vorgesehene Rollen geführt und durch eine Spannvorrichtung gespannt gehalten werden. Damit soll neben einer rein passiven Behandlung oder Anwendung einer Person auch die Möglichkeit gegeben werden, durch selbst initiierte Körperbewegungen eine Anwendung auf Grund von Gleit- und Reibwirkung durchzuführen. Die Wanne kann mit einem Zu- und einem Ablauf versehen sein, durch die z.B. Warmluft oder Warmwasser, Duftstoffe und andere Ingredienzien in den Wannenraum unterhalb der Folie eingeleitet werden können. Verschiedene Anwendungsstoffe und Substanzen, die fließfähig, cremeartig, pastös usw. sein können, sollen auf die Folie aufgebracht werden können und sich auf der sich leicht schalenförmig ausbildenden Auflagefläche besonders zum deren tiefsten Punkt fließen oder gleiten. Diese Vorrichtung ist nur für bestimmte Anwendungen geeignet. Um die Notwendigkeit einer gründlichen Reinigung der Folie nach jeder Anwendung zu umgehen, wird vorgeschlagen, die Folie aus hygienischen Gründen als Einmalfolie auszubilden; sie muss dann nach jeder Anwendung ausgewechselt werden und eine neue Folie muss in die Verspannung an der Wanne für die nächste Anwendung eingeführt werden. Sowohl eine gründliche Reinigung als auch das Auswechseln der Folie ist umständlich und zeitaufwendig. Die Verwendung von Einmalfolien steht im Widerspruch zum Gedanken des Umweltschutzes und der Abfallvermeidung.

In WO 2010/005274 wird ein weiteres Beispiel für eine bekannte Liege offenbart.

Um in einer physiotherapeutischen Praxis oder einem Wellness-Studio die unterschiedlichsten Behandlungen am menschlichen Körper sowohl im Trocken- wie im Nassbereich anbieten zu können, werden sonach verschiedene, auf die verschiedenen Anwendungen oder Behandlungen ausgelegte Arten von Liegen mit entsprechendem Platzbedarf als Ausstattung benötigt, wobei eine "Wanne" mit unter den Begriff "Liege" fallen soll.

Aufgabe der Erfindung ist es, eine Liege für die physiotherapeutische Behandlung des menschlichen Körpers zu schaffen, die sich für die unterschiedlichsten Behandlungen sowohl im Trocken- als auch im Nassbereich eignet, so dass z.B. in einer kleinen Praxis nur noch eine solche Liege für alle angebotenen Behandlungen benötigt wird, der Platzbedarf also erheblich reduziert ist. Sie muss rutschsicher sein und nach jeder Anwendung oder Behandlung leicht und in kurzer Zeit hygienisch einwandfrei zu reinigen sein. Die zu behandelnde Person soll immer eine entspannte, bequeme Stellung einnehmen können, und auch die behandelnde Person soll ihre Arbeit in einer möglichst schonenden, wenig belastenden Haltung ausführen können.

Dies wird erfindungsgemäß mit einer Liege nach Anspruch 1 erreicht.

Die Liegefläche wird durch eine auf dem Unterbau ruhende Platte gebildet, die mindestens aus drei Schichten, nämlich einer Trägerplatte, einer darauf aufgebrachten Estrich-Schicht, in die ein Wärmespender eingebettet ist, und der auf der Estrich-Schicht aufgebrachten, eigentlichen Liegefläche besteht, wobei die Liegefläche mosaikartig aus miteinander verfugten, flachen Bruchsteinstücken zusammengesetzt ist. Als Material für die verwendeten Bruchsteinstücke kommt jede Art von Naturstein, wie Bruchmarmor, Stäbchenmarmor, gebrochener Schiefer, aber auch Fliesen vorzugsweise in Bruchstücken in Frage. Durch die verfugten Bruchsteinstücke kommt eine rutschsichere Liegefläche zu Stande, die durch den unter ihr in der Estrichschicht eingebetteten Wärmespender erwärmt werden kann und leicht und schnell hygienisch einwandfrei zu reinigen ist. Der erwärmte Naturstein vermittelt der auf der Liegefläche Platz nehmenden Person ein angenehmes Gefühl, und es erübrigt sich ein besonderer Überzug. Die Verwendung von Bruchsteinstücken hat außerdem den Vorteil, dass solches Material verhältnismäßig preisgünstig zur Verfügung gestellt wird. Auf Grund des verwendeten Materials kann die Liege für alle erdenklichen Behandlungen und Anwendungen sowohl im Trocken- als auch im Nassbereich eingesetzt werden. Besondere Liegen für den Trocken- und den Nassbereich sind nicht mehr erforderlich. Somit ergibt sich eine erhebliche Platzerspamis, die besonders für kleinere Behandlungsräume von Vorteil ist.

Der Wärmespender kann aus einer elektrischen Heizspirale mit Temperaturregelung oder aus einer Rohrspirale mit Warmwasserdurchlauf bestehen, wobei die Temperatur regelbar ist.

Die Temperatur der Liegefläche wird während des Betriebs vorzugsweise durchgehend auf einer Bereitschaftstemperatur von ca. 15° gehalten und für eine Behandlung vorübergehend auf 38° (Körpertemperatur) erwärmt. Damit lässt sich die Liege auf energie- und kostensparende Weise in Bereitschaft halten und kann nach einer vorübergehenden Unterbrechung der Behandlungen in kurzer Zeit wieder einsatzbereit sein.

Verteilhaft ruht die Platte auf einem wannenförmigen Unterbau und ist am Rand des Unterbaus und durch im Unterbau angeordnete Stützen abgestützt.

Indem am Unterbau ein Wasseranschluss mit Warmwasser-Mischbatterie und Duschschlauch mit Duschkopf vorgesehen ist, wird die Liege auch für Anwendungen im Nassbereich optimiert.

Vorteilhaft ist im Fußende-Bereich der Platte ein Wasserabfluss vorgesehen, der mit einem in dem Unterbau befindlichen Abflussrohr verbunden ist. Die Arbeit an der Liege im Nassbereich wird damit sehr erleichtert.

Wenn zwischen Kopfende und Fußende der auf der Platte ausgebildeten Liegefläche ein Gefälle von ca. 5 cm besteht, kann sich bei der Behandlung verwendete Flüssigkeit zwangsläufig am Wasserabfluss sammeln und abfließen. Es entsteht keine unnötige Nässe im Behandlungsraum.

Wenn sich das Gefälle nicht über die gesamte Länge der Liegefläche erstreckt, sondern nur über deren Drittel am Fußende ausgebildet ist, ist dies für eine auf der Liegefläche liegende Person angenehm, da der Körper dann nicht die Tendenz hat, in Richtung Fußende zu rutschen.

Am Kopfende des Unterbaus kann eine in ihrer Neigung verstellbare Kopfstütze vorgesehen sein; die Verstellung kann manuell oder hydraulisch erfolgen

Als Zusatzvorrichtung kann eine an der Liegefläche rundum abschließende, bewegliche Dampfhaube vorgesehen werden, in die in einem Wasserbehälter erzeugter Dampf eingeleitet werden kann; die Dampfhaube weist dabei eine Aussparung für den Kopf einer auf der Liegefläche liegenden Person auf. Damit werden auf der erfindungsgemäßen Liege auch Dampfbehandlungen möglich.

Der wannenförmige Unterbau kann aus Beton oder Ytongsteinen bestehen und mit einer Verkleidung aus mosaikartig zusammengesetzten, flachen Bruchsteinstücken versehen sein.

Der wannenförmige Unterbau kann auf einem Sockel stehen. Wenn ein Unterbau nach der Erfindung in einer bestimmten Höhe vorgefertigt ist, kann er mit Hilfe des Sockels auf eine erwünschte Höhe gebracht und angepasst werden.

Wo eine ortsfeste Installation der Liege nicht möglich oder nicht erwünscht ist, kann der Unterbau auch aus einem Eisenbett oder aus einem höhenverstellbaren Eisengestell bestehen. Die Liege wird damit beweglich und an verschiedenen Orten einsetzbar.

Die Erfindung wird im Folgenden anhand der anhängenden Zeichnung beispielhaft näher beschreiben; es zeigen
- Fig. 1: eine bevorzugte Ausführungsform der erfindungsgemäßen Liege in perspektivischer Darstellung,
- Fig. 2: die Liege nach Fig. 1, wobei die eigentliche Liegefläche mit ihren Komponenten und der Unterbau getrennt dargestellt sind,
- Fig. 3: einen Querschnitt durch die Liegefläche und ihre Komponenten nach der Ausführungsform gemäß Fig. 1 und Fig. 2,
- Fig. 4: in einer perspektivischen Darstellung den Unterbau und die einzelnen Komponenten, aus denen sich die Liegefläche nach einer bevorzugten Ausführungsform zusammensetzt,
- Fig. 5: eine perspektivische Darstellung einer erfindungsgemäßen Liege mit Zusatzvorrichtung für eine Dampfbehandlung,
- Fig. 6: eine Seitenansicht der Liege nach Fig. 5 und
- Fig. 7: einen Querschnitt durch die Liege nach Fig. 5.

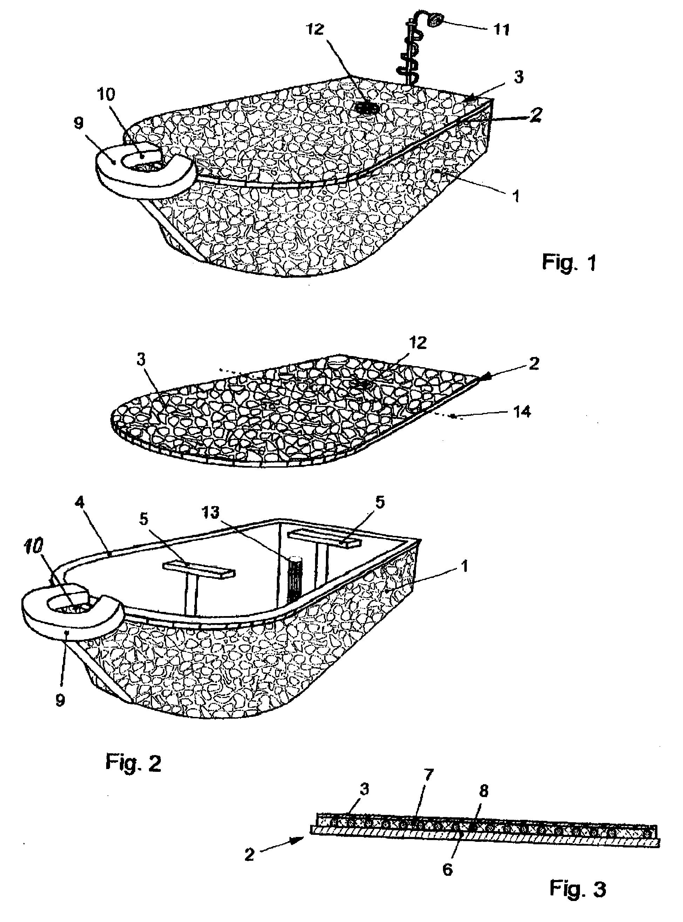

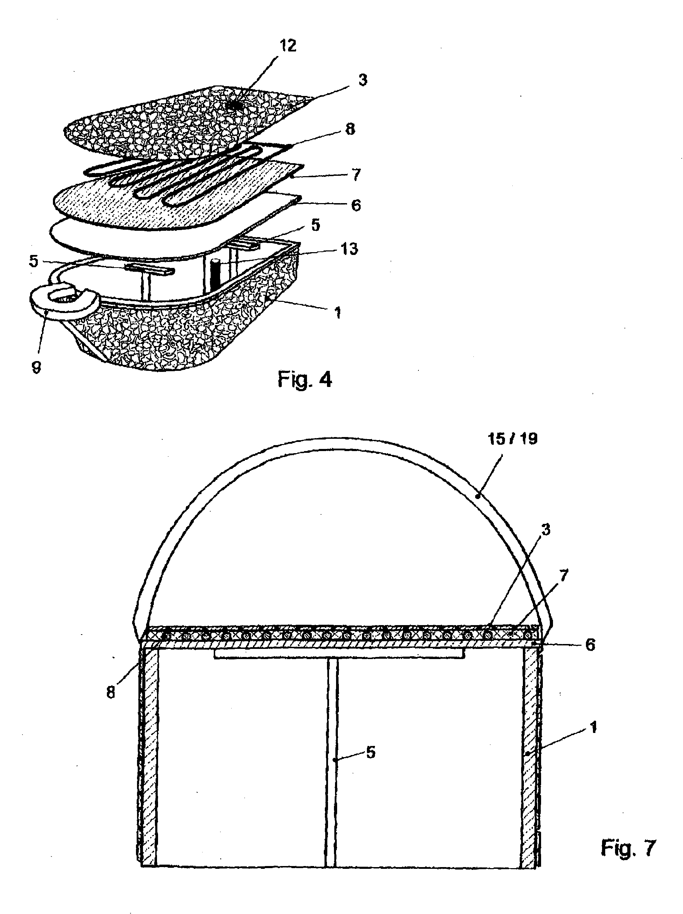

Gemäß Fig. 1 besteht die erfindungsgemäße Liege aus einem vorzugsweise wannenförmigen Unterbau 1 von ca. 2 m Länge und mindestens 80 cm Breite, der mit einer an den Umriss der Öffnung des Unterbaus 1 angepassten Platte 2 abgedeckt ist. Die Platte 2 besteht aus mehreren miteinander verbundenen Schichten (siehe Fig. 3 und Fig. 4), und auf ihr ist die Liegefläche 3 für eine physiotherapeutisch zu behandelnde Person ausgebildet. Die Platte 2 liegt zum einen auf dem Rand 4 des Unterbaus 1 auf und wird im Innern des wannenförmigen Unterbaus 1 außerdem von Stützen 5 getragen (siehe Fig. 2). Als Stützen 5 können z.B. 3 bis 4 Ytong-Steine in den Unterbau 1 eingesetzt werden. Andere Arten von Stützen sind möglich. Die Liegefläche 3 als oberste Schicht der Platte 2 besteht aus mosaikartig zusammengesetzten, miteinander verfugten, unterschiedlich großen Bruchsteinplatten oder Bruchsteinplättchen; dafür können verschiedenste Materialien, vorzugsweise Naturstein zur Anwendung kommen, wie Bruchmarmor, Stäbchenmarmor, gebrochener Schiefer, Fliesen, auch diese können gebrochen sein, u. a. Die einzelnen Bruchsteinplättchen können eine Dicke von ca. 3 bis 4 mm haben. Die Verwendung von Bruchstein für die Liegefläche 3 hat nicht nur den Vorteil, dass solches Material zu einem günstigen Preis zur Verfügung gestellt wird, noch wichtiger für die Erfindung ist, dass die Liegefläche 3 durch die Fugen zwischen den einzelnen mosaikartig zusammengesetzten Steinen rutschsicher wird. Denn, um Rutschgefahr zu vermeiden, soll die Liegefläche 3 keinesfalls als glatte Oberfläche ausgebildet sein. Der Abrieb, vor allem für den Nassbereich, soll nicht unter 12 liegen, (für Fliesen für ein normales Bad ist ein Abrieb von 9 vorgeschrieben).

Die unterste Schicht der Platte 2 wird von einer Trägerplatte 6, z.B. in Form eines Betongerüsts, gebildet (siehe Fig. 3 und Fig. 4). Auf die Trägerplatte 6 ist als Mittelschicht ein Estrich 7 aufgebracht, in den ein beheizbarer Wärmespender 8, z.B. in Form einer elektrischen Heizspirale 8 oder eines Warmwasserdurchlaufs, eingebettet ist; auf dem Estrich 7 mit Wärmespender 8 liegt die mosaikartig zusammengesetzte Liegefläche 3 als oberste Schicht. Durch den Wärmespender 8 ist die Liegefläche 3 erwärmbar, die Temperatur ist regelbar, eine Temperatur von ca. 38° wird als besonders angenehm bei verschiedenen Anwendungen am menschlichen Körper empfunden. Für besondere Anwendungen kann auch eine höhere Temperatur notwendig sein. Das Heizsystem für den Wärmespender 8 kann Energie sparend ausgeführt werden, indem die Temperatur durchgehend auf 15° gehalten wird und für eine Behandlung innerhalb von 30 Minuten auf 38° Körpertemperatur aufgeheizt wird. Der auf Körpertemperatur erwärmte Naturstein der Liegefläche 3 vermittelt einer auf der Liegefläche liegenden Person ein angenehmes Gefühl, ein besonderer textiler Überzug ist nicht erforderlich. Es werden Wäsche und Waschmittel eingespart.

Am Kopfende des Unterbaus 1 ist eine an sich bekannte Kopfstütze 9 vorgesehen, die in ihrer Neigung verstellbar ist. Sie ist aus einer Nullstellung in der Ebene der Liegefläche 3 sowohl nach oben als auch nach unten um jeweils ca. 30° verstellbar, damit eine auf der Liegefläche 3 liegende Person sowohl in Rücken- als auch in Bauchlage eine bequeme Haltung einnehmen kann. Die Kopfstütze 9 weist dazu außerdem eine Öffnung 10 auf, an deren Rand der Kopf in jeder Lage abgestützt ist, die in Bauchlage der Person aber das Gesicht frei lässt. Die Verstellung der Kopfstütze 9 kann manuell-mechanisch oder bevorzugt hydraulisch erfolgen. Als Material für die Kopfstütze 9 empfiehlt sich Kunstleder, auf jeden Fall sollte es wasserfest sein.

Die Liege ist vorzugsweise in unmittelbarer Nähe eines Wasseranschlusses mit einer Warm- und Kaltwasser-Mischbatterie aufgestellt oder fest installiert, an die ein Duschkopf 11 mit einem vorteilhaft mindestens 3,5m langen Duschschlauch angeschlossen ist. Nahe am Fußende der Liege ist in der die Liegefläche 3 bildenden Platte 2 ein Wasserablauf 12 vorgesehen, der, wenn die Platte 2 auf den Unterbau 1 aufgelegt ist, Verbindung mit einem Abflussrohr 13 im Unterbau 1 hat (siehe auch Fig. 2 und Fig. 4). Die Liegefläche 3 kann zum Fußende hin leicht abfallend, um ca. 5cm, gestaltet sein, so dass sich bei einer physiotherapeutischen Behandlung im Nassbereich verwendetes Wasser von selbst am Fußende im Bereich des Wasserablaufs 12 sammelt und abfließen kann. Das Gefälle muss sich nicht über die gesamte Länge der Liegefläche 3 erstrecken, sondern kann ungefähr auf das Drittel der Länge, in der sich der Wasserablauf 12 befindet beschränkt sein, wie es in Fig. 2 durch die gestrichelte Linie 14 angedeutet ist. Eine auf der Liegefläche 3 Platz nehmende Person kann dann mit dem Rumpf auf einer geraden Ebene liegen, nur die Beine liegen auf der Schräge; dies ist für die Person insofern bequemer, als der Körper dann nicht die Tendenz hat, in Richtung Fußende zu rutschen.

Der Unterbau 1 kann aus Beton, Betonteilen oder Ytonsteinen bestehen und kann mit Naturstein, mit Fliesen, Marmor oder ähnlichem Material verkleidet sein, vorzugsweise kann auch dafür Bruchmaterial zur Anwendung kommen. Er sollte eine angemessene Höhe haben, um einerseits ein bequemes Platznehmen einer zu behandelnden Person zu ermöglichen, andererseits auch die Arbeit einer behandelnden Person zu erleichtern oder wenigstens nicht zu erschweren. Die Höhe des Unterbaus 1 kann beispielsweise am Kopfende etwa 70 cm und am Fußende etwa 65 cm betragen. Der Unterbau 1 kann auch auf einem zusätzlichen Sockel stehend ausgebildet sein, mit dem sich dann bei der Einrichtung eine andere bestimmte Höhe der Liege erreichen lässt.

Falls eine ortsfeste Installation der Liege mit einem Unterbau 1 aus Beton nicht möglich oder nicht erwünscht ist, kann der Unterbau 1 auch in Form eines beweglichen Eisenbettes oder eines beweglichen und höhenverstellbaren Gestells bereitgestellt werden. Die Liege würde damit insgesamt beweglich. Auf jeden Fall soll der Unterbau 1 in welcher Form auch immer er ausgeführt wird, wasserfest sein.

Die erfindungsgemäße Liege ist insgesamt auf Grund der verwendeten Materialien wasserfest und leicht und schnell zu reinigen. Sie braucht nach jeder Anwendung nur, eventuell unter Zugabe eines Reinigungs- und/oder Desinfektionsmittels, mit Wasser abgespritzt zu werden. Eine erwünschte Trocknung der Liegefläche 3 kann über den in die Platte 2 integrierten Wärmespender 8 ohne weitere Maßnahmen ebenso schnell erfolgen. Die Liege eignet sich so für die verschiedensten Anwendungen am menschlichen Körper sowohl im Trocken- als auch im Nassbereich, also Massagen, Kosmetikbehandlungen, Körperpackungen, Peelings, Aromatherapiebehandlungen zum Öffnen der Poren vor einer Massage oder Packung, Ayurveda-Anwendungen, Anti-Cellulite-Behandlungen, Seifenbürstenmassagen und andere.

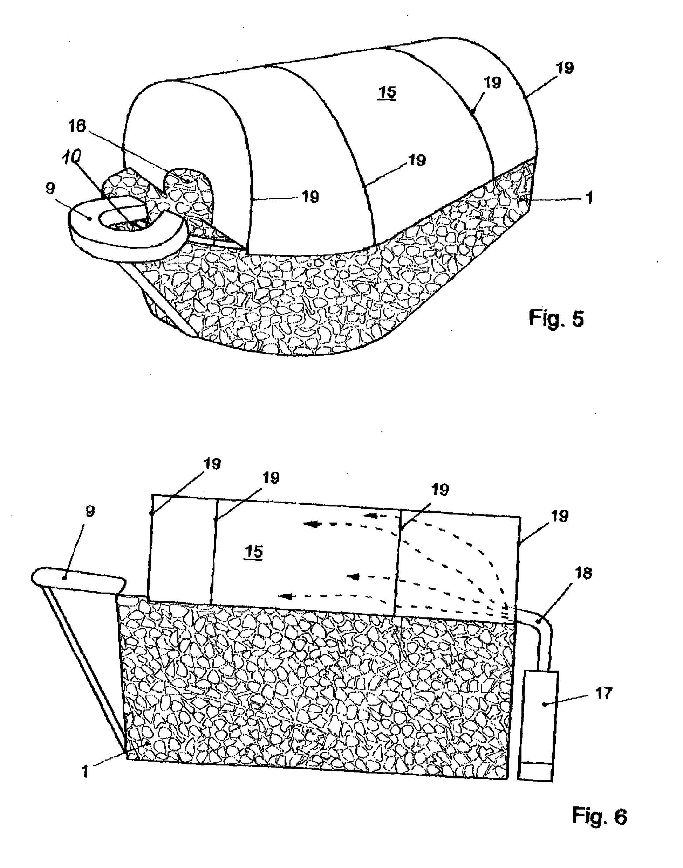

Die erfindungsgemäße Liege eignet sich auch für Dampfbehandlungen mit Sauna- und Schwitz-Effekt. Dazu wird auf die Liegefläche 3 und die darauf liegende Person eine Dampfhaube 15 gestülpt, wie es in den Fig. 5 bis 7 dargestellt ist. Die Dampfhaube 15 reicht vom Fußende der Liege bis nahe ans Kopfende bzw. die Kopfstütze 9 und schließt seitlich und am Fußende am Rand der Liegefläche 3 bzw. an der Außenwand des Unterbaus 1 ab; am Kopfende schließt die Dampfhaube 15 auf der Liegefläche 3 ab und weist hier eine Aussparung 16 für den Kopf einer Person auf. Neben der Liege, vorzugsweise an ihrem Fußende, befindet sich ein Wasserbehälter 17, in dem Dampf erzeugt werden kann, der dann durch einen Schlauch 18 unter die Dampfhaube 15 geleitet wird (siehe Fig. 6). Die Dampfhaube 15 kann aus Segeltuch oder einer Zeltplane bestehen, die durch Streben 19 gestützt und in Form gehalten wird. Die Dampfhaube 15 schließt, wie bereits erwähnt, am Rand der Liegefläche 3 bzw. an der Wand des Unterbaus 1 ab, damit während der Behandlung möglicht kein oder möglichst wenig Dampf und Wärme verloren geht.

Die erfindungsgemäße Liege eignet sich für alle denkbaren physiotherapeutischen Behandlungen sowohl im Trocken- wie im Nassbereich und im Wellnessbereich. Eine physiotherapeutische Praxis benötigt also nur noch mindestens eine solche Liege, um die verschiedensten Behandlungen und Anwendungen auf kleinstem Raum anbieten zu können; der Mindest-Platzbedarf hierfür beträgt nur ca. 4 x 4m². Die leichte und schnelle Reinigung der Liege nach jeder Anwendung führt auch zu einer erheblichen Zeitersparnis bei der praktischen Arbeit in einer Praxis.

### Bezugszeichenliste:

- 1: Unterbau
- 2: Platte
- 3: Liegefläche
- 4: Rand
- 5: Stützen
- 6: Trägerplatte
- 7: Estrich
- 8: Wärmespender, Heizspirale
- 9: Kopfstütze
- 10: Öffnung
- 11: Duschkopf
- 12: Wasserablauf
- 13: Abflussrohr
- 14: gestrichelte Linie
- 15: Dampfhaube
- 16: Aussparung
- 17: Wasserbehälter
- 18: Schlauch
- 19: Streben

## Patentansprüche

1. Liege zur physiotherapeutischen Behandlung des menschlichen Körpers mit einer auf einem Unterbau ruhenden, erwärmbaren Liegefläche, **dadurch gekennzeichnet, dass** die Liegefläche (3) durch eine auf dem Unterbau (1) ruhende Platte (2) gebildet wird, die mindestens aus drei Schichten, nämlich einer Trägerplatte (6), einer darauf aufgebrachten Estrich-Schicht (7), in die ein Wärmespender (8) eingebettet ist, und der auf der Estrich-Schicht (7) aufgebrachten, eigentlichen Liegefläche (3) besteht, wobei die Liegefläche (3) mosaikartig aus miteinander verfugten, flachen Bruchsteinstücken zusammengesetzt ist.

2. Liege nach Anspruch 1, **dadurch gekennzeichnet, dass** der Wärmespender (8) aus einer elektrischen Heizspirale (8) mit Temperaturregelung besteht.

3. Liege nach Anspruch 1, **dadurch gekennzeichnet, dass** der Wärmespender (8) aus einer Rohrspirale mit Warmwasserdurchlauf besteht, wobei die Temperatur des Warmwassers regelbar ist.

4. Liege nach Anspruch 2 oder 3, **dadurch gekennzeichnet, dass** die Temperatur der Liegefläche während des Betriebs durchgehend auf einer Bereitschaftstemperatur von ca. 15° haltbar ist und für eine Behandlung vorübergehend auf 38° (Körpertemperatur) erwärmbar ist

5. Liege nach Anspruch 1, **dadurch gekennzeichnet, dass** die Platte (2) auf einem wannenförmigen Unterbau (1) ruht und am Rand des Unterbaus (1) und durch im Unterbau (1) angeordnete Stützen (5) abgestützt ist.

6. Liege nach Anspruch 5, **dadurch gekennzeichnet, dass** am Unterbau (1) ein Wasseranschluss mit Warmwasser-Mischbatterie und Duschschlauch mit Duschkopf (11) vorgesehen ist.

7. Liege nach Anspruch 5, **dadurch gekennzeichnet, dass** im Fußende-Bereich der Platte (2) ein Wasserabfluss (12) vorgesehen ist, der mit einem in dem Unterbau (1) befindlichen Abflussrohr (13) verbunden ist.

8. Liege nach Anspruch 1 oder 5, **dadurch gekennzeichnet, dass** zwischen Kopfende und Fußende der auf der Platte (2) ausgebildeten Liegefläche (3) ein Gefälle von ca. 5 cm besteht.

9. Liege nach Anspruch 8, **dadurch gekennzeichnet, dass** das Gefälle sich nicht über die gesamte Länge der Liegefläche (3) erstreckt, sondern nur über deren Drittel am Fußende ausgebildet ist.

10. Liege nach Anspruch 1, **dadurch gekennzeichnet, dass** am Kopfende des Unterbaus (1) eine in ihrer Neigung verstellbare Kopfstütze (9) vorgesehen ist.

11. Liege nach Anspruch 10, **dadurch gekennzeichnet, dass** die Kopfstütze (9) in ihrer Neigung manuell oder hydraulisch verstellbar ist.

12. Liege nach Anspruch 1, **gekennzeichnet durch** eine an der Liegefläche (3) rundum abschließende, bewegliche Dampfhaube (15), in die in einem Wasserbehälter (17) erzeugter Dampf einleitbar ist, wobei die Dampfhaube (15) eine Aussparung (16) für den Kopf einer auf der Liegefläche (3) liegenden Person aufweist.

13. Liege nach Anspruch 5, **dadurch gekennzeichnet, dass** der wannenförmige Unterbau (1) aus Beton oder Ytongsteinen besteht und mit einer Verkleidung aus mosaikartig zusammengesetzten, flachen Bruchsteinstücken versehen ist.

14. Liege nach Anspruch 5, **dadurch gekennzeichnet, dass** der wannenförmige Unterbau (1) auf einem Sockel ruht.

15. Liege nach Anspruch 1, **dadurch gekennzeichnet, dass** der Unterbau (1) aus einem Eisenbett besteht.

16. Liege nach Anspruch 1, **dadurch gekennzeichnet, dass** der Unterbau (1) aus einem höhenverstellbaren Eisengestell besteht.

## Claims

1. Bed for the physiotherapeutic treatment of the human body, comprising a heatable bed surface resting on a substructure, **characterized in that** the bed surface (3) is formed by a plate (2) which rests on the substructure (1) and consists of at least three layers, namely a carrier plate (6), a screed layer (7) which is mounted on the latter and in which a heat dispenser (8) is embedded, and the actual bed surface (3) which is mounted on the screed layer (7), wherein the bed surface (3) is composed in the manner of a mosaic from flat rubble stone pieces grouted together.

2. Bed according to Claim 1, **characterized in that** the heat dispenser (8) consists of an electric heating coil (8) with temperature control.

3. Bed according to Claim 1, **characterized in that** the heat dispenser (8) consists of a pipe coil through which hot water passes, wherein the temperature of the hot water is controllable.

4. Bed according to Claim 2 or 3, **characterized in that** the temperature of the bed surface during operation is maintainable continuously to a standby temperature of approx. 15° and is temporarily heatable to 38° (body temperature) for a treatment.

5. Bed according to Claim 1, **characterized in that** the plate (2) rests on a tub-shaped substructure (1) and is supported on the edge of the substructure (1) and by props (5) arranged in the substructure (1).

6. Bed according to Claim 5, **characterized in that** plumbing to a hot water mixing faucet and shower hose with a shower head (11) is provided on the substructure (1).

7. Bed according to Claim 5, **characterized in that** a water outlet (12) which is connected to an outlet pipe (13) located in the substructure (1) is provided in the foot end region of the plate (2).

8. Bed according to Claim 1 or 5, **characterized in that** there is a slope of approx. 5 cm between the head end and foot end of the bed surface (3) formed on the plate (2).

9. Bed according to Claim 8, **characterized in that** the slope does not extend over the entire length of the bed surface (3) but rather is formed only over a third thereof at the foot end.

10. Bed according to Claim 1, **characterized in that** a head rest (9) which is adjustable in the inclination thereof is provided at the head end of the substructure (1).

11. Bed according to Claim 10, **characterized in that** the head rest (9) is adjustable in the inclination thereof manually or hydraulically.

12. Bed according to Claim 1, **characterized by** a movable steam hood (15) which is sealed all the way around on the bed surface (3) and into which steam produced in a water tank (17) is introducible, wherein the steam hood (15) has a recess (16) for the head of a person lying on the bed surface (3).

13. Bed according to Claim 5, **characterized in that** the tub-shaped substructure (1) consists of concrete or Ytong stones and is provided with a cladding made of flat rubble stone pieces assembled in the manner of a mosaic.

14. Bed according to Claim 5, **characterized in that** the tub-shaped substructure (1) rests on a base.

15. Bed according to Claim 1, **characterized in that** the substructure (1) consists of an iron bed.

16. Bed according to Claim 1, **characterized in that** the substructure (1) consists of a height-adjustable iron frame.

## Revendications

1. Couchette pour le traitement physiothérapeutique du corps humain avec une surface de couchage chauffante, reposant sur une infrastructure, **caractérisée en ce que** la surface de couchage (3) est formée par une plaque (2) reposant sur l'infrastructure (1), qui se compose d'au moins trois couches, à savoir une plaque de support (6), une couche de garnissage (7) déposée sur celle-ci, dans laquelle un distributeur de chaleur (8) est noyé, et la surface de couchage (3) proprement dite, déposée sur la couche de garnissage (7), dans laquelle la surface de couchage (3) est composée à la manière d'une mosaïque avec des fragments de pierre plats joints les uns aux autres.

2. Couchette selon la revendication 1, **caractérisée en ce que** le distributeur de chaleur (8) se compose d'un serpentin de chauffage électrique (8) avec régulation de la température.

3. Couchette selon la revendication 1, **caractérisée en ce que** le distributeur de chaleur (8) se compose d'un serpentin tubulaire avec circulation d'eau chaude, dans laquelle la température de l'eau chaude peut être régulée.

4. Couchette selon la revendication 2 ou 3, **caractérisée en ce que** la température de la surface de couchage peut être maintenue en continu pendant le fonctionnement à une température de marche d'environ 15°C et peut être portée temporairement à 38°C (température corporelle) pour un traitement.

5. Couchette selon la revendication 1, **caractérisée en ce que** la plaque (2) repose sur une infrastructure en forme de cuvette (1) et est supportée sur le bord de l'infrastructure (1) et par des appuis (5) disposés dans l'infrastructure (1).

6. Couchette selon la revendication 5, **caractérisée en ce qu'**il est prévu sur l'infrastructure (1) un raccordement d'eau avec une batterie de mélange d'eau chaude et un tuyau de douche avec un pommeau de douche (11).

7. Couchette selon la revendication 5, **caractérisée en ce qu'**il est prévu dans la région d'extrémité de pied de la plaque (2) une évacuation d'eau (12), qui est raccordée à un tuyau d'évacuation (13) se trouvant dans l'infrastructure (1).

8. Couchette selon la revendication 1 ou 5, **caractérisée en ce qu'**il existe une dénivellation d'environ 5 cm entre l'extrémité de tête et l'extrémité de pied de la surface de couchage (3) formée sur la plaque (2).

9. Couchette selon la revendication 8, **caractérisée en ce que** la dénivellation ne s'étend pas sur toute la longueur de la surface de couchage (3), mais uniquement sur le tiers de celle-ci à l'extrémité de pied.

10. Couchette selon la revendication 1, **caractérisée en ce qu'**il est prévu à l'extrémité de tête de l'infrastructure (1) un appuie-tête (9) d'inclinaison réglable.

11. Couchette selon la revendication 10, **caractérisée en ce que** l'appuie-tête (9) présente une inclinaison réglable de façon manuelle ou hydraulique.

12. Couchette selon la revendication 1, **caractérisée par** une hotte à vapeur mobile (15), se formant tout autour de la surface de couchage (3), dans laquelle de la vapeur produite dans un réservoir d'eau (17) peut être introduite, dans laquelle la hotte à vapeur (15) présente une découpe (16) pour la tête d'une personne étendue sur la surface de couchage (3).

13. Couchette selon la revendication 5, **caractérisée en ce que** l'infrastructure en forme de cuvette (1) est constituée de béton ou de blocs Ytong et est dotée d'un recouvrement en fragments de pierre plats, assemblés à la manière d'une mosaïque.

14. Couchette selon la revendication 5, **caractérisée en ce que** l'infrastructure en forme de cuvette (1) repose sur un socle.

15. Couchette selon la revendication 1, **caractérisée en ce que** l'infrastructure (1) se compose d'un lit en fer.

16. Couchette selon la revendication 1, **caractérisée en ce que** l'infrastructure (1) se compose d'un châssis de fer réglable en hauteur.
